# FASCICULE DE BREVET EUROPEEN

(11) **EP 1 458 349 B1**
(45) Date de publication et mention de la délivrance du brevet: **05.11.2014**
(21) Numéro de dépôt: 02805813.9
(22) Date de dépôt: 27.12.2002
(51) Int. Cl.: A61Q 19/00, A61K 8/49, A61K 31/42

(54) **COMPOSITION COMPRENANT AU MOINS UNE OXAZOLINE POUR INHIBER LA MIGRATION DES CELLULES DE LANGERHANS,ET SES UTILISATIONS**
MINDESTENS EIN OXAZOLIN ENTHALTENDE ZUSAMMENSETZUNG ZUR INHIBIERUNG DER MIGRATION DER LANGERHANSZELLEN, UND DEREN VERWENDUNG
COMPOSITION COMPRISING AT LEAST AN OXAZOLIN FOR INHIBITING LANGERHANS CELL MIGRATION, AND USES THEREOF

(30) Priorité: 27.12.2001 FR 0116917
(43) Date de publication de la demande: 22.09.2004
(73) Titulaire: Laboratoires Expanscience, 92400 Courbevoie (FR)
(72) Inventeur: MSIKA, Philippe, F-78000 Versailles (FR); PICCARDI, Nathalie, F-38120 Saint Egrève (FR); PICCIRILLI, Antoine, F-78000 Versailles (FR)
(74) Mandataire: Regimbeau
(86) Numéro de dépôt international: PCT/FR2002/004583
(87) Numéro de publication internationale: WO 2003/055463

(56) Documents cités:
- US-A- 4 876 249
- US-A- 5 360 811

## Description

La présente invention se rapporte au traitement pharmaceutique, notamment dermatologique de la peau. Plus particulièrement, la présente invention concerne l'utilisation d'une composition contenant au moins un composé actif choisi parmi les oxazolines, éventuellement en association avec au moins un autre composé tel qu'un inhibiteur de métalloprotéases, un inhibiteur de PKC, un agent anti-inflammatoire, un agent apaisant, un immunosuppresseur, un chélateur d'ions, une oxazolidinone, un dérivé d'acide carbamique ou un alcanolamide, pour les traitements objets des revendications.

L'invention a également pour objet une telle composition pour son utilisation comme médicament dans les traitements objets des revendications avantageusement pour inhiber la migration des cellules telles que les dendrocytes dermiques, les monocytes, les lymphocytes, et notamment les cellules de Langerhans suite par exemple à un stimulus extérieur ou « signal danger » d'origine chimique, physique, biologique et plus particulièrement immunitaire, dont l'intensité serait suffisamment importante pour induire une perturbation de l'homéostasie cutanée. Les oxazolines, ainsi que leur association avec un inhibiteur de métalloprotéases, un inhibiteur de PKC, un agent anti-inflammatoire, un agent apaisant, un immunosuppresseur, un chélateur d'ions, une oxazolidinone, un dérivé d'acide carbamique ou un alcanolamide et les compositions pharmaceutiques les comprenant sont utiles pour la préparation de médicaments destinés au traitement ou à la prévention des pathologies cutanées d'origine allergique et/ou inflammatoire et/ou irritative et/ou de l'inconfort cutanée (peaux sensibles, réactives ou intolérantes).

La description décrit également une méthode de traitement cosmétique des peaux et/ou des muqueuses sensibles, irritées, intolérantes, à tendance allergique, âgées, soumises à un signal danger, présentant un trouble de la barrière cutanée, présentant des rougeurs cutanées, ou présentant un trouble, un déséquilibre ou un désordre immunologique non pathologique, consistant à appliquer sur la peau et/ou les muqueuses une telle composition.

Une des principales fonctions de la peau est la protection de l'organisme contre des agressions du milieu extérieur. Cette protection est assurée en grande partie grâce à la coopération de cellules présentes dans la peau, cellules qui sont capables en présence d'un agent nocif de générer une réponse inflammatoire et/ou immunitaire dirigée contre l'agent nocif. Ce sont les cellules dendritiques, les cellules de Langerhans (CL) de l'épiderme, et dendrocytes dermiques, les monocytes, les lymphocytes, les kératinocytes, les mastocytes, et les cellules endothéliales vasculaires.

Les CL sont des cellules dendritiques issues de la moelle épinière et qui résident dans les tissus non lymphoïdes tels que la peau et les muqueuses (bouche, poumon, vessie, rectum, vagin). Dans la peau, les CL s'intercalent entre les kératinocytes épidermiques en position suprabasale. Sur le plan ultrastructural, elles sont caractérisées par la présence d'un organite spécifique d'origine membranaire, le granule de Birbeck. Sur le plan immunohistochimique, les CL expriment notamment la molécule CD1a et les molécules du Complexe Majeur d'Histocompatibilité de classe II.

Les CL jouent un rôle déterminant dans l'immunité, en tant que cellules présentatrices de l'antigène. En effet, des expériences menées chez la souris démontrent que les CL capturent les antigènes présents au niveau de l'épiderme et migrent vers les tissus lymphoïdes drainants la peau, où elles présentent l'antigène aux cellules T. L'initiation de la réponse immune cutanée dépend de la capacité des CL à quitter l'épiderme pour migrer jusqu'aux ganglions proximaux. Différents facteurs peuvent influencer cette migration : l'expression de molécules d'adhérence, les protéines de la matrice extracellulaire, des haptènes, des cytokines, etc. Néanmoins, les mécanismes impliqués dans la migration des CL ne sont pas totalement encore élucidés. En particulier, avant d'atteindre les ganglions lymphatiques, les CL doivent non seulement traverser la jonction dermo-épidermique (JDE) mais également se frayer un chemin au travers de la matrice extracellulaire (MEC) dermique. La JDE est principalement composés de laminine 5, de collagène de type IV et VII, de nidogène et de perlecan. La MEC qui entoure les fibroblastes du derme contient essentiellement des collagènes de type I et III.

La maturation, ainsi que l'initiation et la régulation de la migration des CL sont sous la dépendance de cytokines pro-inflammatoires telles que l'IL-1β (interleukine-1-beta) et le TNF-α (Tumor Necrosis-alpha). Il en résulte que toute agression cutanée, plus particulièrement toute réaction inflammatoire et/ou irritative, capable d'induire en quantité suffisante l'une ou l'autre de ces cytokines ou les deux, est capable de stimuler la migration des CL, et donc de faciliter la réaction allergique si ces CL sont associées à un antigène.

Des pathologies de type dermatologique peuvent être observées comme résultant de la migration des CL suite à la capture d'un antigène de surface. Dans l'eczéma atopique, les CL sont capables de fixer des IgE en surface et d'induire une réponse immunitaire pathologique. Dans l'eczéma de contact, les CL jouent un rôle central puisqu'elles captent et traitent l'antigène avant de le présenter aux lymphocytes T. Celui-ci va le garder en mémoire et la réaction immunitaire sera déclenchée au deuxième contact.

Compte tenu de ce qui précède, il est hautement souhaitable de pouvoir modifier la capacité migratoire des dendrocytes dermiques, des monocytes, des lymphocytes, des cellules de Langerhans (CL), pour tenter d'augmenter le seuil de tolérance ou de limiter la réactivité de la peau allergique et/ou inflammatoire et/ou irritée ainsi que de la peau atopique et/ou sensible et/ou réactive et/ou inconfortable. C'est le problème que se propose de résoudre la présente invention. Les inventeurs ont en effet démontré de manière tout à fait surprenante et inattendue que des composés tels que les oxazolines permettent d'inhiber de manière spectaculaire la migration des cellules, telles que les cellules de Langerhans notamment induite par la présence d'un agent allergène.

Les oxazolines forment une classe particulière de composés dont les applications sont connues depuis fort longtemps (J.A. Frump, Chemical Reviews, 1971, vol. 71, n°5, pp 484-505). Ces composés sont en effet utilisés comme agents de revêtements, agents protecteurs de surfaces, agents stabilisants, dispersants, notamment des ions métalliques, comme agents plastifiants, tensio-actifs, inhibiteurs de corrosion, agents anti-mousse ou encore comme additifs dans les huiles minérales lubrifiantes et les adhésifs. Par ailleurs, les oxazolines sont aussi connues pour leurs propriétés antimicrobiennes et antifongiques et sont utilisées à ce titre comme agents conservateurs. Sur le plan pharmocologique, les oxazolines présentent des propriétés diverses telles qu'une action régulatrice du système nerveux central (tranquillisants), une action anti-dépressive, une action vasoconstrictive, une action coupe-faim, une action inhibitrice des acétylcholinostérases, et une action sédative. Cependant, les oxazolines n'ont jamais été décrites dans l'art antérieur comme capables d'inhiber la migration des cellules de Langerhans notamment induite par la présence d'un agent allergène.
Le brevet US 4 876 249 décrit des compositions thérapeutiques comprenant un principe actif et à titre d'excipient pour favoriser la pénétration du principe actif une oxazoline. Le brevet US 5 360 811 décrit des 2-amino-1,3-propanediols comprenant des groupements oxazolines et des groupements thiényles.

La présente invention concerne ainsi l'utilisation d'un composé actif pour inhiber la migration des cellules de Langerhans choisi dans le groupe des oxazolines. Avantageusement, la composition est une composition cosmétique ou pharmaceutique, notamment dermatologique, comprenant au moins un excipient cosmétiquement ou pharmaceutiquement acceptable. Les oxazolines selon l'invention répondent aux formules générales suivantes: 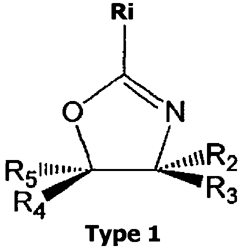 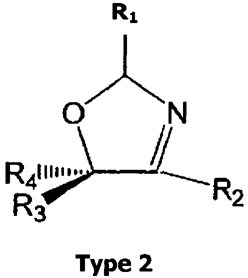 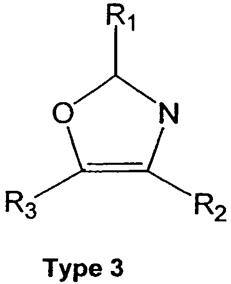 dans laquelle R₁ représente un groupe alkyle en C₁-C₄₀, linéaire ou ramifié, saturé ou insaturé, comprenant éventuellement une ou plusieurs insaturation(s) éthylénique(s) ainsi que un ou plusieurs substituant(s) choisi(s) dans le groupe formé par les radicaux hydroxy (OH) et alcoxy en C₁-C₆ (OC₁-C₆), R₂, R₃, R₄ et R₅ représentent, de manière indépendante, un atome d'hydrogène, un radical hydroxy, ou un groupe alkyle en C₁-C₃₀, linéaire ou ramifié, saturé ou insaturé, comprenant éventuellement une ou plusieurs insaturations éthyléniques ainsi que un ou plusieurs substituant(s) choisi(s) dans le groupe formé par les radicaux hydroxy (OH), alcoxy en C₁-C₆ (OC₁-C₆) et alcoxy en C₁-C₆ carbonyles (COOC₁-C₆). Par le terme de "alcoxy en C₁-C₆ (OC₁-C₆)", on entend au sens de la présente invention, un radical alcoxy dont le groupement alkyle comprend de 1 à 6 atomes de carbone.

De préférence, la dite oxazoline est une oxazoline de type 1 sélectionnée dans le groupe composé de la 2-undécyl-4-hydroxyméthyl-4-méthyl-1,3-oxazoline, de la 2-undécyl-4,4-diméthyl-1,3-oxazoline, de la (E)-4,4-diméthyl-2-heptadéc-8-ényl-1,3-oxazoline, de la 4-hydroxyméthyl-4-méthyl-2-heptadécyl-1,3-oxazoline, la (E)-4-hydroxyméthyl-4-méthyl-2-heptadéc-8-ényl-1,3-oxazoline, la 2-undécyl-4-éthyl-4-hydroxyméthyl-1,3-oxazoline. De manière plus préférée, la dite oxazoline est la 2-undécyl-4,4-diméthyl-1,3-oxazoline appelée OX-100 de formule:

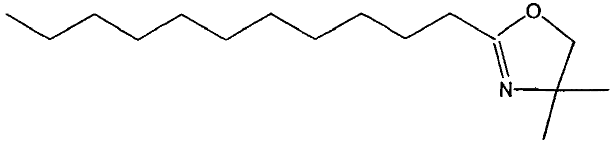

De nombreuses voies de synthèse sont connues pour préparer les composés oxazolines selon l'invention. Ainsi, celles-ci peuvent être préparées par synthèse chimique en faisant réagir un acide gras (ou un ester méthylique) et un amino-alcool, le plus souvent en présence d'un agent azéotropique afin de favoriser l'élimination de l'eau formée (et du méthanol formé). Une autre voie de synthèse possible consiste à condenser un halo-amide en présence d'une base forte ou de carbonate de sodium (R. M. Lusskin, J. Amer. Chem. Soc., 72, (1950), 5577). Les oxazolines peuvent également être synthétisées par réaction des époxydes sur les nitriles, par réaction du chlorure de thionyle sur les hydroxyamides ou encore, par action d'un acide sur une aziridinylphosphine.

Selon un mode de réalisation de l'invention, la composition peut comprendre en outre au moins un inhibiteur de la migration des cellules de Langerhans sélectionné dans le groupe des inhibiteurs de métalloprotéases matricielles (MMPs).

Par « composés inhibiteurs des métalloprotéases matricielles (MMPs) » on entend selon l'invention tout composé connu de l'homme du métier pour sa capacité d'inhiber l'activité de dégradation de la matrice extracellulaire par les MMPs. Les MMPs constituent une famille d'enzymes (actuellement plus d'une vingtaine ont été identifiées et caractérisées), zinc-dépendantes, de structure très conservée, qui possèdent la capacité de dégrader les composants de la matrice extracellulaire. Elles sont classées selon la nature de leur substrat en collagénases, gélatinases et stromélysine. Elles peuvent être synthétisées par différents types cellulaires au niveau de la peau (fibroblastes, kératinocytes, macrophages, cellules endothéliales, éosinophiles, cellules de Langerhans, etc.). Le groupe des MMPs est ainsi constitué de quatre sous-classes : (1) les collagénases, (2) les gélatinases, (3) les stromelysines et (4) les MMP de type membranaires (MT-MMPs). L'activité des MMPs peut être modulée par des inhibiteurs de protéinase naturellement présents tels que les inhibiteurs tissulaires de métalloprotéinases (TIMPs ; notamment les TIMP-1 et TIMP-2). En particulier, le composé actif pour inhiber la migration des cellules de Langerhans est un composé inhibiteur d'au moins une MMP choisie dans le groupe constitué par les MMP-1, MMP-2, MMP-3 MMP-9, MMP-7, MMP-13 et MMP-18. Comme « composé inhibiteur des MMPs », en tant que composé actif pour inhiber la migration des cellules de Langerhans selon la présente invention, on entend en particulier les inhibiteurs tissulaires de métalloprotéinases (TIMPs), l'alpha-2-macroglobuline, les inhibiteurs de l'activateur du plasminogène, les chélateurs de zinc, la bryostatine-1, les antibiotiques (doxycyclines, minocyclines, etc.), les peptides synthétiques ou naturels ayant une structure similaire aux substrats des MMPs (batimastat, marimastat, etc.), les rétinoïdes (en particulier les rétinoïdes non aromatiques tels que le rétinaldéhyde, la trétinoïne, et l'acide rétinoïque 9-cis, la vitamine A, les rétinoïdes monoaromatiques tels que l'étrétinate, l'all-trans acitretine et le motrerinide, et les rétinoïdes polyaromatiques tels que l'adapalène, le tazarotène, le tamibarotène et l'arotinoïde methyl sulfone), les antioxydants (les piègeurs d'oxygène singulet, etc.), les anti-cancéreux (ou « anti-métastatiques »), les hydrolysats de malt tels que Colalift commercialisés par la société Coletica, les extraits d'algues marines tels que Kelpadélie commercialisés par la société Secma, les extraits de cartilage de requin tels que le complexe MDI commercialisés par la société Atrium, les peptides de riz comme par exemple Colhibin commercialisé par la société Pentapharm, les extraits peptidiques de lupin. Plus particulièrement, le composé inhibiteur des MMPs selon la présente invention est choisi dans le groupe constitué par les extraits peptidiques de lupin ou « peptides de lupin », tels que ceux décrits dans la demande de brevet FR-99 04 875 déposée le 19 avril 1999 au nom de la société Laboratoires Pharmascience. On peut notamment citer l'extrait peptidique décrit dans la demande FR 99 04875 sous la dénomination extrait B (LU105). Selon un autre mode préféré de réalisation, le dit inhibiteur des MMPs est choisi dans le groupe constitué par les rétinoïdes.

Selon un mode de réalisation particulier de l'invention, la composition peut également comprendre en outre au moins un composé choisi dans le groupe constitué par les inhibiteurs de PKC, les agents anti-inflammatoires, les agents apaisants, les immunosuppresseurs, les chélateurs d'ions, les oxazolidinones, les dérivés d'acide carbamique, notamment le (1-Hydroxyméthyl-tridécyl)-acide carbamique et le (1-Hydroxyméthyl-undécyl)-acide carbamique, et les alcanolamides. Ce ou ces composé(s) choisi(s) dans le groupe constitué par les inhibiteurs de PKC, les agents anti-inflammatoires, les agents apaisants, les immunosuppresseurs, les chélateurs d'ions, les oxazolidinones, les dérivés d'acide carbamique, notamment le (1-Hydroxyméthyl-tridécyl)-acide carbamique et le (1-Hydroxyméthyl-undécyl)-acide carbamique, et les alcanolamides permet(tent) de jouer sur et/ou de limiter la réaction irritative ou de sensibilisation voire pour certains d'entre eux également d'inhiber la migration des cellules dendritiques, plus particulièrement des cellules de Langerhans, des dendrocytes dermiques, des monocytes, des lymphocytes, des kératinocytes, des mastocytes et des cellules endothéliales.

Par « PKC » ou « Protéines Kinases C », on entend au sens de la présente invention les enzymes qui catalysent une réaction de phosphorylation sur un substrat cellulaire.

Lorsqu'elles sont activées, les PKC phosphorylent des résidus sérine ou thréonine spécifiques sur des substrats protéiques, qui varient selon le type cellulaire. Dans de nombreuses cellules, l'activation des PKC augmente la transcription de gènes spécifiques.

Les protéines kinases C (PKC) sont des protéines codées par une famille de gènes (11 isoformes différentes). On sait notamment que ces protéines sont impliquées dans la transduction de signaux extracellulaires médiés par les facteurs de croissance, les cytokines, ainsi que par un certain nombre d'autres molécules biologiques. La protéine kinase β2 (PKC-β2) apparaît exprimée spécifiquement par les CL de l'épiderme.

Tout composé connu de l'homme du métier comme inhibant l'activité de phosphorylation des PKC peut ainsi être utilisé en tant que composé inhibiteur des PKC selon la présente invention. On peut par exemple citer les polypeptides décrits dans la demande WO 99/43805 (Incyte Genomics Inc.).

En particulier, le composé inhibiteur des PKC est choisi dans le groupe constitué par les inhibiteurs non spécifiques des PKC, les inhibiteurs spécifiques de l'isoforme PKC-β2, et les associations de ceux-ci.

Plus particulièrement, le composé inhibiteur des PKC est choisi dans le groupe constitué par les composés phénoliques et polyphénoliques, les procyanidines (catéchines, épicatéchines, etc.), l'alpha-amyrine, le lupéol, le lupéol linoléate, les stérols, les stanols, les alcools triterpéniques et leurs homologues hydrogénés, les antibiotiques tels que la staurosporine, Ro-318425 (ou 2-(8)-(aminométhyl)-6,7,8, 9-tétrahydropyridol(1,2-a)indol-3-yl)3-(1-méthyl-indol-3-ylmaléimide, HCl) tel que commercialisé par la société Calbiochem, les composés qui agissent par compétition avec les activateurs physiologiques des PKC tels que le diacylglycérol et le phorbol ester, les lipides cutanés de type (lyso)sphingolipides, lysophospholipides tels que les céramides et pseudocéramides, sphingosines et phytosphingosines, les sphinganines, les dérivés, précurseurs, analogues et homologues de ces composés, d'origine naturelle ou synthétique.

Par « composés phénoliques et polyphénoliques », on entend selon l'invention les phénols simples, les benzoquinones, les acides phénoliques, les acétophénones, les acides phénylacétiques, les acides hydroxycinnamiques, les coumarines et isocoumarines, les chromones, les naftoquinones, les xanthones, les anthraquinones, les flavonoides, les lignanes et néolignanes, les lignines, les chalcones, les dihydrochalcones, les aurones, les flavones, les flavonols, les dihydroflavonols, les flavanones, les flavanols, les flavandiols ou leucoanthocyanidines, les anthocyanidines, les isoflavonoides, les biflavonoides, les proanthocyanidines et les tanins condensés.

Par "stérols", on entend plus particulièrement selon l'invention le stérol, c'est à dire le composé perhydro-1,2-cyclopentanophenanthrène ayant un groupe hydroxyle à la position 3, et les analogues du stérol de formule générale (I) ci-dessous.

Ainsi, de préférence, les stérols utilisables selon l'invention répondent à la formule générale suivante : 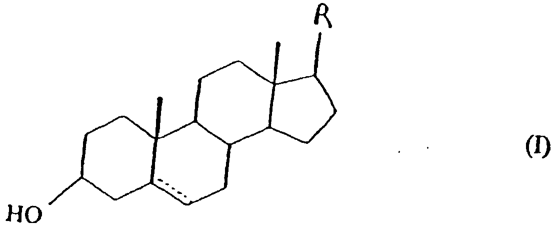 dans laquelle l'insaturation en pointillés en position 5 correspond à l'insaturation dans le cas des stérols, R représente une chaîne hydrocarbonée, linéaire ou ramifiée, insaturée ou non, comportant de 1 à 25 atomes de carbone. En particulier, R est choisi dans le groupe constitué par les groupes alkyle en C₁-C₁₂ les groupes alcoxy en C₁-C₈, les groupes alcényle en C₂-C₈, les groupes alcynyle en C₂-C₈, les groupes cycloalkyle en C₃-C₈, les groupes alcényle en C₂-C₈ halogénés, les groupes alcynyle en C₂-C₈ halogénés. Le terme "halogéné" désigne un ou plusieurs substituant halogène, à savoir un ou plusieurs atome(s) de chlore, fluor, brome ou iode.

Parmi les stérols pouvant être avantageusement utilisés selon l'invention, on peut citer en particulier le β-sitostérol, l'α-sitostérol, le γ-sitostérol, le stigmastérol, le campestérol ou encore le brassicastérol et les mélanges de ceux-ci. Par exemple, le β-sitostérol peut être utilisé sous la forme du produit dénommé "Ultra" (comprenant principalement du β-sitostérol) tel que commercialisé par la société Kaukas. Dans le cas d'une utilisation d'un mélange de stérols, on peut citer par exemple le produit dénommé "Generol" comprenant principalement du β-sitostérol (environ 50 % en poids), du stigmastérol, du brassicastérol et du campestérol tel que commercialisé par la société Cognis ou encore le produit "Primal" de la société Kaukas.

Parmi les alcools triterpéniques pouvant être avantageusement utilisés selon l'invention, on peut citer en particulier la β-amyrine, l'érythrodiol, le taraxastérol, le cycloarténol, le 24-méthylènecycloartanol, le lupéol, le lanostérol et les mélanges de ceux-ci.

Par "homologues hydrogénés" d'un alcool triterpénique, on entend selon l'invention le ou les composés alcool(s) triterpénique(s) correspondant(s) dont la ou les liaison(s) insaturée(s) éventuellement présente(s) ont été hydrogénées (c'est à dire transformée(s) en liaison saturée) selon des méthodes bien connues de l'homme du métier.

Plus particulièrement encore, le composé inhibiteur des PKC est choisi dans le groupe constitué par les sphingolipides et les lysophospholipides, tels que:
- les céramides
- les sphingosines
- les galactocérobrosides
- les psychosines
- les sulfatides
- les lysosulfatides
- les sphyngomyélines, et
- les lysosphingomyélines.

On peut citer également plus particulièrement, comme composé inhibiteur des PKC, les lipides cutanés de type sphingolipides et lysophospholipides.

Comme sphingolipides, on peut citer ceux parmi les plus élémentaires tels que la sphingosine (D érythro dihydroxy 1,3 amino 2 octadécène 4t) et ses isomères, la phytosphingosine (D ribo trihydroxy 1,3,4 amino 2 octadécane) et ses isomères. Mais aussi, les lysosphingolipides (parmi eux, la lysosulfatide et la psychosine), la sulfogalactosylsphingosine, la sphinganine (2-amino 1,3 octadécane diol) et les sphingomyelines.

Comme phospholipides, on peut citer ceux parmi les familles des phosphatidylamino-alcools et des phosphatidylpolyols. Le groupe des phosphatidylamino-alcools comprend notamment les phosphatidylethanolamines (ou phosphatidylcolamines), les phosphatidylcholines, les phosphatidylsérines, les N-acylphosphatidylethanolamines. Celui des phosphatidylpolyols comprend quant à lui, les phosphatidylcholinositols, les diphospho-inositides, les lysodiphospho-inositides, les phosphatidylglycérols et les cardiolipides.

On peut citer également plus particulièrement, comme composé inhibiteur des PKC, les céramides, notamment les céramides du ciment intercornéocytaire de l'épiderme ainsi que les précurseurs des céramides que sont la sphingosine et la phytosphingosine.

D'une manière générale, les céramides peuvent être synthétisés chimiquement (on parle notamment de pseudo-céramides), être d'origine animale (des concentrations relativement élevées de sphingolipides sont présentes dans l'encéphale et la colonne vertébrale des mammifères), d'origine végétale (principalement des cérébrosides et autres sphingolipides glycosylées) ou encore être des dérivés de levures (configuration stéréochimique identique à celle des céramides naturellement présents dans la peau humaine).

Les céramides du ciment intercornéocytaire de l'épiderme peuvent être séparés par les méthodes classiques (chromatographie sur couche mince) en six fractions, correspondant à des composés différant par la nature des acides gras et la nature de la base impliquée (sphingosines, insaturées, ou phytosphingosines, saturées). Le tableau 1 suivant illustre les structures respectives présentes dans ces fractions, selon la classification de Werts et Downing. La fraction 6 peut elle-même être subdivisée par des méthodes plus fines en deux entités : les céramides 6a et 6b.

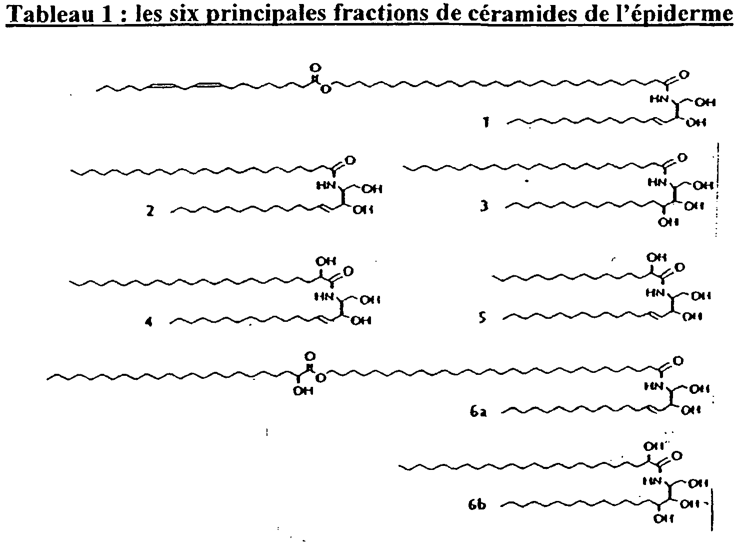

Ainsi, les céramides 1, les moins polaires, comportent une structure tout à fait particulière, que l'on retrouve dans le céramide 6a : un oméga-hydroxyacide à longue chaîne amidifiant la base, et attaché à son extrémité omega par une liaison ester à un autre acide gras (0-acylcéramides). Dans le cas de la fraction 1, les acides gras liés à la sphingosine sont essentiellement en C24, C26, C30, C32 ou C34, pouvant être saturés, monoéthyléniques (principalement pour les C30, C32 et C34) ou diéthyléniques (C32 et surtout C34). Quant à l'acide gras attaché à l'extrémité oméga du précédent, il s'agit, de façon largement prédominante pour les céramides 1, de l'acide linoléique, le rôle capital dans la fonction de barrière hybride de l'épiderme est bien connu.

La fraction 2, de structure plus classique (sphingosines ou dihydrosphingosines liées par une liaison amide à un acide gras, principalement C20 à C28), est la plus abondante.

La fraction 3 est assez similaire, la différence portant sur la nature de la base, qui, en l'occurrence, est essentiellement représentée par les phytosphingosines, saturées.

Les fractions 4 et 5 sont essentiellement caractérisées par la présence d'alpha-hydroxyacides liés à une sphingosine.

La fraction 6b est proche des fractions 4 et 5, comportant un alpha hydroxyacide, mais lié à une phytosphingosine, saturée.

La fraction 6a, comme le céramide 1, comporte le motif caractéristique que l'on ne retrouve qu'au niveau des céramides de l'épiderme, c'est-à-dire la liaison ester entre l'hydroxyle en oméga d'un acide gras lié à une sphingosine, et le groupement carboxylique d'un acide gras terminal, qui, cette fois, n'est pas l'acide linoléique, mais un alpha-hydroxyacide.

Il convient également de citer les phytocéramides (céramides à base phytosphingosine), les cholestérol-céramides synthétiques, les galacto ou gluco cérébrosides.

Enfin, parmi les composés inhibiteurs des PKC pouvant être utilisés selon la présente invention, la sphingosine est présente à l'état naturel dans la peau, et joue entre autre un rôle important dans fonction barrière du *stratum corneum,* en tant que précurseur des sphingolipides (céramides et sphingoglycolipides). Elle peut être dérivée de source biologique telle que des extraits de cerveaux bovins ou par voie de synthèse, à partir de la serine, comme décrit par exemple dans l'article de Newman, J.Am. CHEM., 95 (12) : 4098 (1973). On peut plus particulièrement citer les formes isomères de la sphingosine : D-érythro, L-thréo, L-érythro et D-thréo. La forme D-erythro est la plus fréquemment présente dans la nature.

Selon la présente invention, les composés inhibiteurs des PKC pouvant être utilisés comprennent les isomères, les dérivés (sels, complexes, etc.), les analogues, les homologues, les précurseurs et les métabolites des composés inhibiteurs des PKC décrits ci-dessus.

Les agents anti-inflammatoires pouvant être utilisés dans le cadre de la présente invention en association avec les oxazolines sont des agents anti-inflammatoires non stéroïdiens (AINS).

Les agents apaisants pouvant être utilisés dans le cadre de la présente invention en association avec les oxazolines sont avantageusement des dérivés de réglisse et des dérivés de l'alpha-bisabolol.

Les immunosuppresseurs pouvant être utilisés dans le cadre de la présente invention en association avec les oxazolines sont avantageusement le tacrolimus, le pimécrolimus, et la cyclosporine.

Les chélateurs d'ions pouvant être utilisés dans le cadre de la présente invention en association avec les oxazolines sont avantageusement des chélateurs chimiques avantageusement choisis dans le groupe constitué par l'acide éthylènediamine-tétracétique (EDTA) et ses sels de sodium, de potassium, de calcium disodium, de diammonium, de triéthanololamine (TEA-EDTA), l'acide hydroxyéthyl éthylène diamine tétracétique (HEDTA) et son sel trisodique, l'acide diéthylènetriamine pentacétique (DTPA), et leurs mélanges. Les chélateurs d'ions peuvent également être des chélateurs biologiques avantageusement choisis dans le groupe constitué par la métallothionéine, la transférine, la lactoférine, la calmoduline, le chitosane méthylène phosphonate, et leurs mélanges.

Les ions chélatés sont avantageusement les ions Na⁺, K⁺, Ca²⁺, Cl⁻, Ni²⁺, Co⁺, Co²⁺, Zr ²⁺, Zr⁴⁺, mais aussi les ions chrome au niveau d'oxydation II et III tels que Cr²⁺, Cr³⁺, et Cr₂O₇²⁻.

Les alcanolamides pouvant être utilisés dans le cadre de la présente invention en association avec les oxazolines sont avantageusement des alcanolamides répondant à la formule générale suivante: 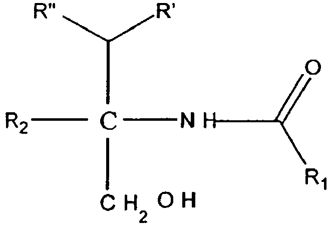 dans laquelle R₁ représente un groupe alkyle en C₁-C₄₀ comprenant de 0 à 6 insaturations et comprenant de manière éventuelle au moins un substituant choisi dans le groupe formé par les radicaux hydroxyles (OH), les alkoxy en C₁-C₆ (OC₁-C₆) et les alkoxy en C₁-C₆ carbonyles (COOC₁-C₆);
R' et R" représentent, de manière indépendante, un atome d'hydrogène, un groupe méthyle, un groupe hydroxyle, un groupe alkyle en C₂-C₂₀ comprenant de 0 à 6 insaturations et comprenant de manière éventuelle au moins un substituant choisi dans le groupe formé par les radicaux hydroxyles (OH), et les alkoxy en C₁-C₆ (OC₁-C₆), à la condition que lorsque R' représente un groupe hydroxyle, R" représente un hydrogène ou un groupe alkyle en C₁-C₆ comprenant de 0 à 3 insaturations et, de manière éventuelle, au moins un substituant choisi dans le groupe formé par les radicaux hydroxyles (OH), les alkoxy en C₁-C₆ (OC₁-C₆) et les alkoxy en C₁-C₆ carbonyles (COOC₁-C₆);
R₂ représente un atome d'hydrogène, un groupe méthyle, un groupe alkyle en C₂-C₂₀ comprenant de 0 à 6 insaturations et comprenant de manière éventuelle au moins un substituant choisi dans le groupe formé par les radicaux hydroxyles (OH), les alkoxy en C₁-C₆ (OC₁-C₆) et les alkoxy en C₁-C₆ carbonyles (COOC₁-C₆).

Par le terme de "alcoxy en C₁-C₆ (OC₁-C₆)", on entend au sens de la présente invention, un radical alcoxy dont le groupement alkyle comprend de 1 à 6 atomes de carbone.

Avantageusement, le radical R₁ représente un groupe alkyle linéaire saturé comprenant de 2 à 40 atomes de carbone (en C₂-C₄₀), de manière avantageuse 6 à 22 atomes de carbone (en C₆-C₂₂), de manière encore plus avantageuse de 8 à 18 atomes de carbone (en C₈-C₁₈), et de manière encore plus avantageuse de 10 à 16 atomes de carbone (en C₁₀-C₁₆).

Dans un autre mode de réalisation de l'invention, le radical R₁ représente un groupe alkyle comprenant de 1 à 40 atomes de carbone (en C₁-C₄₀), avantageusement de 2 à 40 atomes de carbone (en C₂-C₄₀), de manière préférée 6 à 22 atomes de carbone (en C₆-C₂₂) et de manière encore plus préférée de 8 à 18 atomes de carbone (en C₈-C₁₈), comprenant de 1 à 6 insaturations, et comprenant de manière éventuelle au moins un radical hydroxyle, alkoxy ou alkoxycarbonyle tels que définis ci-dessus.

Selon un mode de réalisation de l'invention, R' et R" représentent, de manière indépendante, un atome d'hydrogène, un groupe méthyle, un groupe alkyle linéaire saturé en C₂-C₂₀.

Selon un autre mode de réalisation de l'invention, R₂ représente un atome d'hydrogène, un groupe méthyle ou un groupe alkyle linéaire saturé en C₂-C₂₀.

Selon une variante avantageuse de l'invention, ledit alcanolamide est l'alcanolamide appelé AK100 de formule :

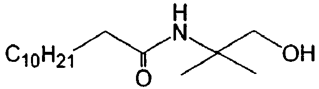

Les dérivés d'acide carbamique pouvant être utilisés dans le cadre de la présente invention en association avec les oxazolines sont avantageusement des dérivés d'acide carbamique répondant aux formules générales suivantes: 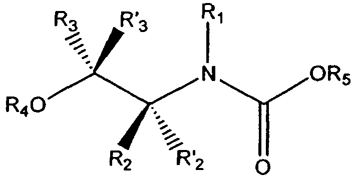 dans laquelle :
R₁ représente un atome d'hydrogène ou un groupe alkyle en C₁-C₃₀, linéaire ou ramifié, saturé ou insaturé, comprenant éventuellement une ou plusieurs insaturation(s) éthylénique(s) et/ou acétylénique(s), ainsi que un ou plusieurs substituant(s) hydroxy (OH);
R₂ et R'₂ représentent, de manière indépendante, un atome d'hydrogène ou un groupe alkyle en C₁-C₃₀, linéaire ou ramifié, saturé ou insaturé, comprenant éventuellement une ou plusieurs insaturation(s) éthylénique(s) et/ou acétylénique(s), ainsi que un ou plusieurs substituant(s) hydroxy (OH);
R₃ et R'₃ représentent, de manière indépendante, un atome d'hydrogène ou un groupe alkyle en C₁-C₃₀, linéaire ou ramifié, saturé ou insaturé, comprenant éventuellement une ou plusieurs insaturation(s) éthylénique(s) et/ou acétylénique(s), ainsi que un ou plusieurs substituant(s) hydroxy (OH);
R₄ et R₅ représentent, de manière indépendante, un atome d'hydrogène ou un groupe acyle de type RxCO, dans lequel Rx est un radical alkyle en C₁-C₃₀, linéaire ou ramifié, saturé ou insaturé, comprenant éventuellement une ou plusieurs insaturation(s) éthylénique(s) et/ou acétylénique(s), ainsi que un ou plusieurs substituant(s) hydroxy (OH).

Selon un mode de réalisation, R₁ représente un atome d'hydrogène.

Selon un autre mode de réalisation, R₂ représente un groupe alkyle linéaire saturé en C₈-C₂₂, avantageusement en C₉-C₁₈, de manière encore plus avantageuse en C₉-C₁₃, de manière encore plus avantageuse en C₁₁-C₁₃, et/ou R'₂ représente un atome d'hydrogène.

Selon un autre mode de réalisation, R₃ et R'₃ représentent un atome d'hydrogène.

Selon un autre mode de réalisation, R₄ et R₅ représentent un atome d'hydrogène.

Dans un mode de réalisation particulier, R₁ représente un atome d'hydrogène, R₂ représente un groupe alkyle linéaire saturé en C₈-C₂₂, avantageusement en C₉-C₁₈, de manière encore plus avantageuse en C₉-C₁₃, de manière encore plus avantageuse en C₁₁-C₁₃, et R'₂, R₃, R'₃, R₄ et R₅ représentent un atome d'hydrogène.

Avantageusement le dérivé d'acide carbamique est choisi dans le groupe constitué par le (1-Hydroxyméthyl-tridécyl)-acide carbamique et le (1-Hydroxyméthyl-undécyl)-acide carbamique.

Le (1-Hydroxyméthyl-tridécyl)-acide carbamique peut être représenté par la formule suivante :

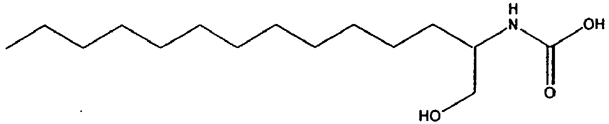

Les oxazolidinones pouvant être utilisées dans le cadre de la présente invention en association avec les oxazolines sont avantageusement des oxazolidinones répondant aux formules générales suivantes: 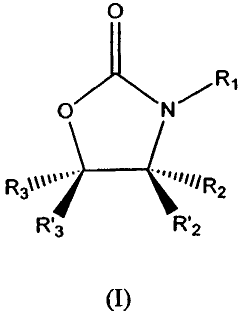 dans laquelle :
R₁ représente un atome d'hydrogène ou un groupe alkyle en C₁-C₃₀, linéaire ou ramifié, saturé ou insaturé, comprenant éventuellement une ou plusieurs insaturation(s) éthylénique(s) et/ou acétylénique(s), ainsi que un ou plusieurs substituant(s) hydroxy (OH);
R₂ et R'₂ représentent, de manière indépendante, un atome d'hydrogène ou un groupe alkyle en C₁-C₃₀, linéaire ou ramifié, saturé ou insaturé, comprenant éventuellement une ou plusieurs insaturation(s) éthylénique(s) et/ou acétylénique(s), ainsi que un ou plusieurs substituant(s) hydroxy (OH);
R₃ et R'₃ représentent, de manière indépendante, un atome d'hydrogène ou un groupe alkyle en C₁-C₃₀, linéaire ou ramifié, saturé ou insaturé, comprenant éventuellement une ou plusieurs insaturation(s) éthylénique(s) et/ou acétylénique(s), ainsi que un ou plusieurs substituant(s) hydroxy (OH).

Selon un mode de réalisation, R₁ représente un atome d'hydrogène.

Selon un autre mode de réalisation, R₂ représente un groupe alkyle linéaire saturé en C₈-C₂₂, avantageusement en C₉-C₁₈, de manière encore plus avantageuse en C₉-C₁₂, de manière encore plus avantageuse en C₁₀-C₁₁, et/ou R'₂ représente un atome d'hydrogène.

Selon un autre mode de réalisation, R₃ et R'₃ représentent un atome d'hydrogène.

Dans un mode de réalisation particulier, R₁ représente un atome d'hydrogène, R₂ représente un groupe alkyle linéaire saturé en C₈-C₂₂, avantageusement en C₉-C₁₈, de manière encore plus avantageuse en C₉-C₁₂, de manière encore plus avantageuse en C₁₀-C₁₁, et R'₂, R₃ et R'₃ représentent un atome d'hydrogène.

Avantageusement l'oxazolidinone est choisie dans le groupe constitué par la 4-dodécyl-oxazolidin-2-one, la 3,4-didodécyl-oxazolidin-2-one et la 4,5-didodécyl-oxazolidin-2-one.

La 4-dodécyl-oxazolidin-2-one peut être représentée par la formule suivante :

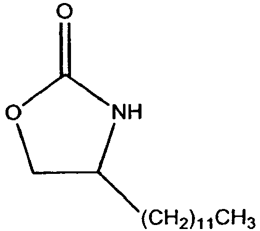

La 3,4-didodécyl-oxazolidin-2-one peut être représentée par la formule suivante:

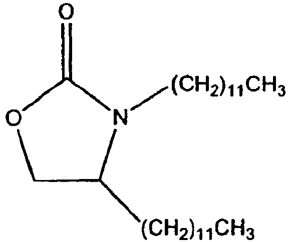

La 4,5-didodécyl-oxazolidin-2-one peut être représentée par la formule suivante :

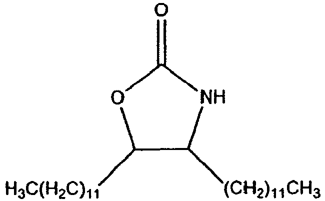

Les alcanolamides, les oxazolidinones et les dérivés d'acides carbamiques sont des composés inhibiteurs de la migration des cellules de Langerhans.

La composition se caractérise en ce que la concentration en oxazoline est avantageusement comprise entre environ 0,001 et environ 10 % en poids, et plus particulièrement entre environ 0,01 et 3 % en poids, par rapport au poids total de la composition.

La composition est avantageusement une composition cosmétique ou pharmaceutique, notamment dermatologique. La composition peut être formulée sous la forme de différentes préparations adaptées à une administration topique, à une administration orale ou rectale, à une administration parentérale. De préférence les différentes préparations sont adaptées à l'administration topique et incluent les crèmes, les pommades, les lotions, les huiles, les patches, les sprays ou tout autres produits pour application externe. Les modes d'administration, les posologies et les formes galéniques optimales des composés et compositions décrits peuvent être déterminés selon les critères généralement pris en compte dans l'établissement d'un traitement cosmétique ou pharmaceutique, de préférence dermatologique, adapté à un patient comme par exemple l'âge ou le poids corporel du patient, la gravité de son état général, la tolérance au traitement, les effets secondaires constatés, le type de peau. En fonction du type d'administration souhaitée, la composition et/ou les composés actifs décrits peuvent en outre comprendre au moins un excipient cosmétiquement acceptable ou pharmaceutiquement acceptable, notamment dermatologiquement acceptable. De préférence, on utilise un excipient adapté pour une administration par voie topique externe. La composition peut en outre comprendre au moins un adjuvant cosmétiquement ou pharmaceutiquement connu de l'homme du métier, choisi parmi les épaississants, les conservateurs, les parfums, les colorants, des filtres chimiques ou minéraux, les agents hydratants, les eaux thermales, etc.

La présente invention concerne l'utilisation d'au moins un composé actif choisi dans le groupe des oxazolines telles que précédemment définies ou d'une composition selon l'invention pour la préparation d'un médicament destiné à inhiber la migration des cellules dendritiques, des dendrocytes dermiques, des monocytes, des lymphocytes, des kératinocytes, des mastocytes et des cellules endothéliales.

Le médicament est destiné à inhiber la migration des cellules de Langerhans.

Ledit médicament est destiné au traitement ou à la prévention des réactions ou pathologies allergiques, et/ou inflammatoires, et/ou irritatives de la peau et/ou des muqueuses, notamment de la bouche, des poumons, de la vessie, du rectum, du vagin.

Avantageusement selon la présente invention, le médicament est destiné au traitement et/ou à la prévention des réactions ou pathologies de la peau et/ou des muqueuses consécutives à la migration des cellules, telles que les cellules de Langerhans, induite par un signal danger. On entend par « signal danger »au sens de la présente invention tout signal entraînant notamment la production de cytokines inflammatoires ou tout signal immunologique vrai du type pénétration d'un allergène.

Selon un mode de réalisation de la présente invention, le médicament est destiné au traitement et/ou à la prévention des réactions ou pathologies induites par des haptènes chimiques ou métalliques.

Selon un autre mode de réalisation de la présente invention, le médicament est destiné au traitement ou à la prévention des peaux et/ou des muqueuses sensibles et/ou réactives et/ou inconfortable et/ou intolérante et/ou présentant un trouble de la barrière cutanée et/ou présentant un déséquilibre immunologique lié au vieillissement intrinsèque, extrinsèque (soleil, pollution) ou hormonal.

En effet, il a été montré que le vieillissement de la peau entraînait une modification du statut immunitaire de celle-ci et que les cellules immunologiques pouvaient modifier leur localisation initiale par suite d'une migration incontrôlée.

La composition décrite, ainsi que les composés actifs décrits permettent de réduire la réponse immunitaire induite par la migration de CL ayant fixé des IgE en surface. C'est pourquoi, la présente invention concerne également l'utilisation d'une composition telle que décrite et destinée à inhiber la migration des cellules de Langerhans ou d'au moins un composé actif choisi dans le groupe des oxazolines telles que définies précédemment pour le traitement ou la prévention de l'eczéma atopique. La composition décrite, ainsi que les composés actifs décrits sont également destinés au traitement ou à la prévention de l'eczéma de contact, dans la mesure où ils permettent de réduire une réponse immunitaire notamment induite par capture d'un antigène, traitement et présentation de cet antigène aux lymphocytes T par les CL.

La composition décrite, ainsi que les composés actifs décrits, sont également utilisés pour le traitement et/ou la prévention de pathologies inflammatoires, notamment de dermatoses inflammatoires telles que le psoriasis, des dermites irritatives, des maladies auto-immunes, de la prévention la photo-immuno- suppression ou du rejet de greffe. Par « photo-immuno-suppression » au sens de la présente invention, on entend désigner la diminution de la réponse immunitaire induite par les ultra-violets solaires, et plus particulièrement par les ultra-violets B.

Enfin, c'est également un des objets de la présente invention d'utiliser une composition décrite ainsi que les composés actifs décrits, pour diminuer le caractère allergisant et/ou irritant d'une composition, qui peut être une préparation pharmaceutique ou une préparation cosmétique, ou d'un parfum. Par caractère allergisant, on entend le pouvoir de certains composés contenus dans les dites préparations de se comporter comme des allergènes, c'est-à-dire des composés capables d'induire une réaction d'hypersensibilité immédiate et/ou inflammatoire.

Dans les différentes utilisations précédemment évoquées du composé actif choisi dans le groupe des oxazolines telles que définies ci-dessus, celui-ci peut-être utilisé en association avec au moins un inhibiteur de la migration des cellules de Langerhans sélectionné dans le groupe constitué par les inhibiteurs de métalloprotéases matricielles (MMPs), les inhibiteurs de PKC, les agents anti-inflammatoires, les agents apaisants, les immunosuppresseurs, les chélateurs d'ions, les oxazolidinones, les dérivés d'acide carbamique et les alcanolamides, tels que définis précédemment.

La composition et les composés actifs décrits sont aussi décrits pour une utilisation en cosmétologie. La présente invention décrit également une méthode de traitement cosmétique des peaux et/ou des muqueuses sélectionnées parmi les peaux et/ou les muqueuses sensibles, irritées, intolérantes, à tendance allergique, âgées, soumises à un signal danger, présentant un trouble de la barrière cutanée, présentant des rougeurs cutanées, ou présentant un désordre ou un déséquilibre immunologique non pathologique lié au vieillissement intrinsèque, extrinsèque ou hormonal, caractérisée en ce qu'elle consiste à appliquer sur la peau et/ou les muqueuses une composition cosmétique selon l'invention ou au moins un composé actif choisi dans le groupe des oxazolines telles que définies précédemment. Le composé actif choisi dans le groupe des oxazolines telles que définies précédemment peut également être appliqué en association avec au moins un autre composé sélectionné dans le groupe constitué par les inhibiteurs de métalloprotéases matricielles (MMPs), les inhibiteurs de PKC, les agents anti-inflammatoires, les agents apaisants, les immunosuppresseurs, les chélateurs d'ions, les oxazolidinones, les dérivés d'acide carbamique et les alcanolamides tels que définis précédemment. Dans le cadre de la méthode de traitement cosmétique, le désordre ou le déséquilibre immunologique non pathologique est un déséquilibre temporaire ou non de la fonction immunitaire de la peau sans caractère de gravité.

D'autres caractéristiques et avantages de l'invention apparaissent dans la suite de la description avec les exemples représentés ci-après. Dans ces exemples on se référera aux figures suivantes. Ces figures et exemples sont destinés à illustrer la présente invention et ne peuvent en aucun cas être interprétés comme pouvant en limiter la portée.

### FIGURES

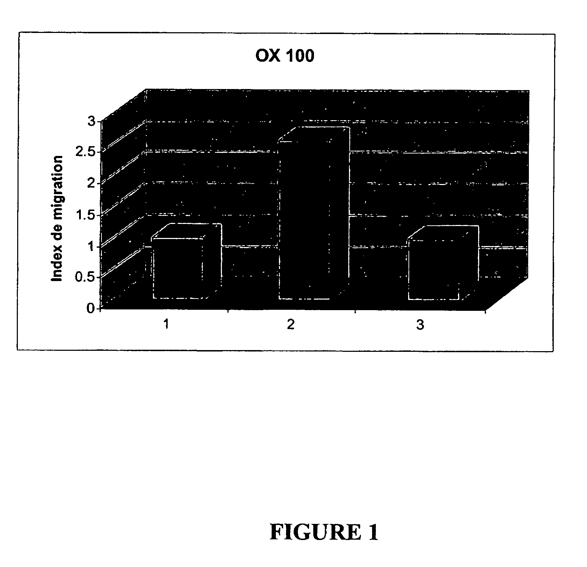

Figure N°1 : Index de migration de cellules de Langerhans fraîchement isolées à partir de peau humaine et activées par du DNSB. Effet de la molécule d'oxazoline OX100 (2-undécyl-4,4-diméthyl-1,3-oxazoline). (1) cellules contrôles; (2) cellules sensibilisées par l'haptène DNSB ; (3) cellules sensibilisées par l'haptène DNSB + OX100 (1 µM).

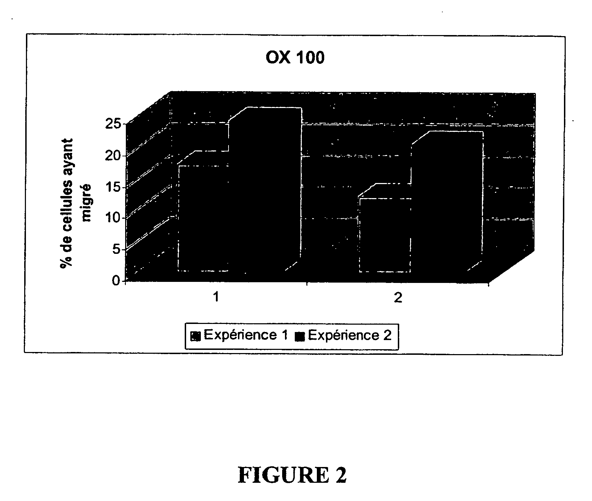

Figure 2 : Pourcentage (%) de migration de cellules dendritiques issues de sang de cordon, après activation par l'haptène. Effet de la molécule d'oxazoline OX100 (2-undécyl-4,4-diméthyl-1,3-oxazoline) .(1) cellules sensibilisées par l'haptène BB ; (2) cellules sensibilisées par l'haptène BB + OX100 (1 µM).

### EXEMPLES

### Exemple 1 : Exemple de composition selon la présente invention

| | % en poids |
|---|---|
| Eau | QSP 100 |
| Polydécène hydrogéné | 8 à 15 |
| Glycérine | 8 à 15 |
| Carbonate de dicaprylyle | 3 à 7 |
| Glucoside de lauryle | 1,5 à 3 |
| Polyglycéryl-2 dipolyhydroxystéarate | 1,5 à 4 |
| Extrait peptidique de lupin (Protéine hydrolysée) | 0,2 à 3 |
| Acrylate/Copolymère d'acrylate d'alkyle en C₁₀-₃₀ | 0,4 |
| Hydroxyméthylglycinate de sodium | 0,4 |
| Gomme de xanthane | 0,3 |
| Oxazoline OX100 | 0,01 à 0,7 |
| Hydroxyde de sodium | 0,07 |
| Acide citrique | 0,03 |

### Exemple 2 : étude de l'activité de l'OX100 sur l'inhibition de la migration des CL fraîchement isolées à partir de fragments de peau humaine

### 1) Matériels & Méthodes

### 1.1 Obtention des cellules de Langerhans

Des suspensions de cellules épidermiques ont été obtenues par traitement enzymatique (0,05% trypsine, pendant 18h à +4°C) de fragments de peau humaine normale issus de chirurgie plastique. Les suspensions obtenues contiennent en moyenne 2 à 4% de CL. L'obtention de suspensions contenant en moyenne 70% de CL, est basée sur le principe de la centrifugation sur gradient de densité (Lymphoprep™) et élimination des kératinocytes.

### 1.2 Préparation des milieux

Le milieu de base choisi pour l'ensemble de l'étude a été le RPMI 1640 (Gibco BRL, France). La molécule d'OX100 fournie par Pharmascience, à la concentration de 10⁻² M en solution dans du DMSO (Dimethyl Sulfoxide), a été diluée en RPMI-1640 et testées à 1 µM.

### 1.3 Sensibilisation des CL

On a utilisé comme agent sensibilisateur le DNSB (Sigma Aldrich), forme soluble du DNCB (dinitro-chloro-benzène), solubilisé en RPMI-BSA et utilisé à la concentration de 50 µM.

### 1.4 Migration des CL

Un système de chambre de culture à deux compartiments (Falcon, Becton Dickinson, France) a été utilisé. Le compartiment supérieur est séparé du compartiment inférieur par une membrane de porosité 8 µm, sur laquelle sont déposées 50 µg/cm² de Matrigel. La membrane est alors recouverte de protéines formant un film équivalent à une membrane basale (laminine, collagène IV, nidogène, entactine, héparane sulfate protéoglycanes). Les cellules reprises dans le milieu RPMI-BSA seul ou en présence des différents produits sont déposées dans le compartiment supérieur. Dans le compartiment inférieur, est ajouté du surnageant de culture de fibroblastes humains normaux. Après 18h d'incubation à 37°C, le nombre de cellules vivantes ayant traversé le Matrigel et se trouvant dans le compartiment inférieur est compté sous microscope (les CL sont facilement identifiables par leur forme dendritique). Chaque essai est réalisé en triplicate.

### 2) Résultats

### 2.1 Les résultats sont présentés dans le tableau 2 suivant et illustrés par l'histogramme de la Figure 1.

**Tableau 2 : index de migration des CL**

| | 1 | 2 | 3 |
|---|---|---|---|
| Index de migration | 1 | 2,55 | 0,98 |

| | | | |
|---|---|---|---|
| *Légende tableau 2 et de l'histogramme de la* *figure 1* *:* 1 : Cellules contrôles 2 : Cellules sensibilisées par l'haptène DNSB 3 : DNSB + OX100 (1 µM) | | | |

### 2.2 Migration des CL

Les résultats représentent le rapport entre le nombre de cellules ayant migré en présence de DNSB +/- OX100 et le nombre de cellules ayant migré dans les conditions normales (cellules contrôles non sensibilisées et non traitées) . Les CL fraîchement isolées de l'épiderme n'ont pas une capacité migratoire élevée. Dans l'expression des résultats, la capacité migratoire des CL contrôles (non traitées et non sensibilisées) est arbitrairement fixée à 1.

Le traitement des cellules avec l'haptène DNSB a stimulé la migration des CL de façon significative (+155%) par rapport aux cellules normales non stimulées (cellules contrôles). L'OX100 à la concentration de 1 µM inhibe de manière significative la migration des CL induite par le DNSB. Les cellules ainsi traitées ont un index de migration comparable à celui des cellules contrôles non sensibilisées.

Les inventeurs ont montré en utilisant des CL fraîchement isolées, placées dans un système de chambre de culture à deux compartiments (permettant la migration cellulaire), que de manière tout à fait surprenante, l'OX100 inhibe de manière significative la migration des CL. Dans les conditions expérimentales utilisées par les inventeurs, les cellules traitées par l'OX100 ont un index de migration comparable à celui des cellules contrôles non sensibilisées.

### Exemple 3: étude de l'activité de l'OX100 sur l'inhibition de la migration des cellules dendritiques générées in vitro à partir de précurseurs CD34+ issus de sang de cordon

### 1) Matériels & Méthodes

### 1.1 Génération de Langerhans-like in vitro

Les cellules mononuclées ont été obtenues à partir de sang de cordon ombilical de donneurs sains, par centrifugation sur Ficoll. Les cellules CD34+ ont ensuite été purifiées par immunosélection à l'aide d'anticorp spécifique et de billes magnétiques (Miltenyi Biotech, Germany). Les cellules CD34+ ont été cultivées en présence de GM-CSF (100 ng/ml), TNF-α (2.5 ng/ml) en RPMI additionné de 10% sérum de veau foetal, pendant 5 jours. L'addition de TGF-ß1, facteur qui favorise la différenciation des cellules vers la voie cellules de Langerhans, a été réalisée au 5ième jour de culture.

### 1.2 Préparation des milieux (Idem exemple 2).

### 1.3 Sensibilisation des CL

Les cellules ont été traitées, au 7ième jour par l'haptène BB (Base de Brandowski, 1.17 µg/ml), pendant 24 h, puis soumises au test de migration.

### 1.4 Migration des CL (Idem exemple 2).

### 2) Résultats

### 2.1 Les résultats de deux expériences indépendantes sont présentés dans le tableau 3 suivant et illustrés par l'histogramme de la Figure 2.

**Tableau 3 : Pourcentage de cellules dendritiques générées in vitro avant migré**

| | 1 | 2 |
|---|---|---|
| Expérience 1 | 17 | 12 |
| Expérience 2 | 24 | 20,3 |

| | | |
|---|---|---|
| *Légende tableau 3 et de l'histogramme de la* *figure 2* *:* 1 : Cellules sensibilisées par l'haptène BB 2 : BB + OX100 (1 µM) | | |

### 2.2 Migration des CL

Les résultats représentent le pourcentage de cellules ayant migré en présence des différents produits testés. Le pourcentage est calculé en rapportant le nombre de cellules récupéré dans le compartiment inférieur de la chambre de migration au nombre de cellules soumises à la migration. Dans les expériences 1 et 2, l'OX100 inhibe respectivement de 29 et 15% la migration des cellules dendritiques.

L'OX100 à la concentration de 1 µM inhibe de manière significative la migration des cellules dendritiques générées *in vitro* et activées par l'haptène BB.

### Exemple 4 : Etude de l'activité de l'oxazoline OX100, seul ou en association avec LU105, sur l'inhibition de la migration des cellules dendritiques sur la souris

### 1. Matériels & méthodes

### 1.1 Réactifs

Le FITC (Fluorescéine isothiocyanate, Sigma, St. Louis, MO) a été dilué extemporanément dans un mélange acétone : dibutylphthalate (1:1).

### 1.2 Inhibiteurs

L'oxazoline OX100 et le LU105 (LU 105 est un inhibiteur de MMPs, correspondant à un extrait peptidique de lupin blanc commercialisé par la Société Expanscience sous le nom de marque Actimp 193®) ont été fournis par les "Laboratoires Expanscience", et formulés seuls ou en association dans un véhicule inerte compatible avec une application topique [oxazoline OX100 (0,1%) ± LU105 (2%)]. Les différentes formulations ont été appliquées sur les oreilles de souris deux fois par jour pendant 4 jours consécutifs. Trois heures après la dernière application, 1,5% de FITC sont appliqués sur les deux oreilles (l'une traitée et l'autre non traitée (Contrôle)).

### 1.3 Migration des cellules de Langerhans (CL) et des cellules dendritiques

### CD sur la souris

L'effet des deux molécules a été évalué in vivo chez la souris. La peau des deux oreilles est badigeonnée avec 1,5% FITC (2X5 µl). 24 h après, les souris sont sacrifiées et une suspension cellulaire est préparée à partir des ganglions auriculaires et cervicaux (ganglions drainants, ci-après dénommés GL) ou à partir des ganglions popliteaux (ganglions non drainants, contrôle négatif). Les tissus ont été découpés et les cellules sont séparées par filtration (filtre 100 µM, Falcon; Becton Dickinson), puis lavés. Les cellules sont ensuite centrifugées 10 min à 600 × g (m x s⁻²) sur gradient de metrizamide (14,5% dans du RPMI 1640 ; 7,5% SVF). Les cellules de l'interface sont récupérées, rincées puis marquées avec un AC anti-CDS86 PE-conjugué, biot-MHC CLII mAbs plus streptavidine-Cya (PharMingen) et analysées par cytométrie en flux. Seules les cellules FITC+, PE+ et Cya+ sont comptabilisées car elles représentent la population de cellules ayant migré de la peau vers les GL suite à l'application de l'haptène.

### 2. Résultats

L'application topique du véhicule seul n'entraîne aucune modification du nombre de CD FITC+ au niveau des GL. Le véhicule est donc sans effet sur les capacités migratoire des CD.

Les résultats portant sur la migration des CD sont présentés dans le tableau 4 suivant.

**Tableau 4 :**

| | OXAZOLINE OX100 (0,1 %) | LU105 (2%) | OXAZOLINE OX100 (0,1%) + LU105 (2%) |
|---|---|---|---|
| Inhibition de la migration en % | 30 | 40 | 90 |

La migration des CD au niveau des GL, suite à l'application de l'haptène FITC, est inhibée dans des proportions comparables et sans différence significative par l'oxazoline OX100 à la dose de 0,1% et le LU105 à la dose de 2%.

Lorsque les deux types de molécules sont associés, cette inhibition est presque totale. En conclusion, il a ainsi été montré de manière tout à fait surprenante, en utilisant un modèle de souris sensibilisées par l'haptène FITC, que l'oxazoline OX100 inhibe de manière significative la migration des CD vers les GL. Par ailleurs, l'oxazoline OX100 et le LU 105 agissent en synergie pour inhiber la migration des CD chez la souris sensibilisée.

### Exemple 5 : Etude de l'activité de l'oxazoline OX100, seule ou en association avec AK100, sur l'inhibition de la migration des cellules dendritiques sur la souris

### 1. Matériels & méthodes

### 1.1 Réactifs

Le FITC (Fluorescéine isothiocyanate, Sigma, St. Louis, MO) a été dilué extemporanément dans un mélange acétone : dibutylphthalate (1:1).

### 1.2 Inhibiteurs

L'oxazoline OX100 et AK100 (décrit précédemment) ont été fournis par les "Laboratoires Expanscience", et formulés seuls ou en association dans un véhicule inerte compatible avec une application topique, [OX100 (0,05%) ± AK100 (0,05%)]. Les différentes formulations ont été appliquées sur les oreilles de souris deux fois par jour pendant 4 jours consécutifs. Trois heures après la dernière application, 1,5% de FITC sont appliqués sur les deux oreilles (l'une traitée et l'autre non traitée (Contrôle)).

### 1.3 Migration des cellules de Langerhans (CL) et des cellules dendritiques CD sur la souris

L'effet des deux molécules a été évalué in vivo chez la souris. La peau des deux oreilles est badigeonnée avec 1,5% FITC (2×5 µl). 24 h après, les souris sont sacrifiées et une suspension cellulaire est préparée à partir des ganglions auriculaires et cervicaux (ganglions drainants, ci-après dénommés GL) ou à partir des ganglions popliteaux (ganglions non drainants, contrôle négatif). Les tissus ont été découpés et les cellules sont séparées par filtration (filtre 100 µM, Falcon; Becton Dickinson), puis lavés. Les cellules sont ensuite centrifugées 10 min à 600 × g (m x s⁻²) sur gradient de metrizamide (14,5% dans du RPMI 1640 ; 7,5% SVF). Les cellules de l'interface sont récupérées, rincées puis marquées avec un AC anti-CDS86 PE-conjugué, biot-MHC CLII mAbs plus streptavidine-Cya (PharMingen) et analysées par cytométrie en flux. Seules les cellules FITC+, PE+ et Cya+ sont comptabilisées car elles représentent la population de cellules ayant migré de la peau vers les GL suite à l'application de l'haptène.

### 2. Résultats

L'application topique du véhicule seul n'entraîne aucune modification du nombre de CD FITC+ au niveau des GL. Le véhicule est donc sans effet sur les capacités migratoire des CD.

Les résultats portant sur la migration des CD sont présentés dans le tableau 5 suivant.

**Tableau 5 :**

| | OX100 (0,05%) | AK100 (0,05%) | OX100 (0,05%) + AK100 (0,05%) |
|---|---|---|---|
| Inhibition de la migration en % | 15 | 15 | 40 |

La migration des CD au niveau des GL, suite à l'application de l'haptène FITC, est inhibée dans des proportions comparables et sans différence significative par OX100 à la dose de 0,05% et AK100 à la dose de 0,05%.

Lorsque les deux types de molécules sont associés, cette inhibition est plus importante. En conclusion, il a ainsi été montré de manière tout à fait surprenante, en utilisant un modèle de souris sensibilisées par l'haptène FITC, que l'oxazoline OX100 inhibe de manière significative la migration des CD vers les GL. Par ailleurs, OX100 et AK100 agissent en synergie pour inhiber la migration des CD chez la souris sensibilisée.

### Exemple 6: Evaluation des effets d'une crème cosmétique de jour comprenant l'oxazoline OX100 pour peau hypersensible, irritée ou à prédisposition allergique

Une crème de jour cosmétique comprenant 0,1% en poids d'OX100 et 2% en poids d'extrait peptidique de lupin blanc, LU105, par rapport au poids totale de la crème, a été testée sur des sujets humains volontaires, avec la collaboration de médecins dermatologues.

Les buts principaux sont d'évaluer l'efficacité clinique et l'acceptabilité cosmétique de ladite crème de jour dans le contexte d'une utilisation normale du produit.

Le produit du test, mis à la disposition du praticien avec les informations publiques nécessaires, a été proposé par le Dermatologue à son patient en précisant des modalités d'applications quotidiennes suffisantes soit au moins 2 applications par jour. Le produit a été appliqué sur le visage matin et soir sur une peau propre et sèche.

Au total, la durée de l'étude pour chaque sujet a été de 4 semaines avec deux observations, relevées avant puis après cette période de 4 semaines d'application.

A la consultation finale, le sujet a été interrogé de façon non directive sur la survenue éventuelle d'effets indésirables locaux.

Les tests, suivant le protocole décrit précédemment, ont été effectués sur 73 femmes dont 37 ont une peau sensible et 36 ont une peau irritée.

Les résultats sont évalués sur une échelle allant de 0 à 9. Une cotation de 0 correspond à une évolution de la peau, avant et après traitement, nulle, une cotation allant de 1 à 3 correspond à une évolution de la peau, avant et après traitement, légère, une cotation allant de 4 à 6 correspond à une évolution de la peau, avant et après traitement, modérée, et une cotation allant de 7 à 9 correspond à une évolution de la peau, avant et après traitement, importante.

Les résultats sont présentés dans le tableau 6 suivant.

**Tableau 6 :**

| ***Evaluation clinique dermatologue*** | | **Echantillon Global** | **Groupe Peau sensible** | **Groupe Peau irritée** |
|---|---|---|---|---|
| | | *(73 femmes)* | *(37 volontaires)* | *(36 volontaires)* |
| Erythème | Note moyenne sur 10 à | 3,89 | 4,16 | 3,61 |
| | Note moyenne sur 10 à J30 | 1,73 | 1,95 | 1,50 |
| | *% évolution de J0 à J30* | -*56*% | *-53%* | *-58%* |
| Sécheresse | Note moyenne sur 10 à J0 | 4,33 | 4,62 | 4,03 |
| | Note moyenne sur 10 à J30 | 1,47 | 1,57 | 1,36 |
| | *% évolution de J0 à J30* | -66% | -66% | -66% |
| Desquamation | Note moyenne sur 10 à J0 | 2,71 | 2,59 | 2,83 |
| | Note moyenne sur 10 à J30 | 0,75 | 0,59 | 0,92 |
| | *% évolution de J0 à J30* | -72% | -77% | *-68%* |
| Oedème | Note moyenne sur 10 à J0 | 0,90 | 1,08 | 0,72 |
| | Note moyenne sur 10 à J30 | 0,10 | 0,11 | 0,08 |
| | *% évolution de J0 à J30* | -89% | *-90%* | *-88%* |
| Vésicules | Note moyenne sur 10 à J0 | 0,40 | 0,73 | 0,06 |
| | Note moyenne sur 10 à J30 | 0,03 | 0,05 | 0,00 |
| | *% évolution de J0 à J30* | *-93%* | -93% | *-100%* |
| Rugosité | Note moyenne sur 10 à J0 | 1,71 | 1,94 | 1,47 |
| | Note moyenne sur 10 à J30 | 0,38 | 0,49 | 0,28 |
| | *% évolution de J0 à J30* | *-78%* | *-75%* | *-81%* |
| | | | | |
| Prurit | Note moyenne sur 10 à J0 | 2,96 | 3,16 | 2,75 |
| | Note moyenne sur 10 à J30 | 0,63 | 0,57 | 0,69 |
| | *% évolution de J0 à J30* | *-79%* | -82% | -*75*% |
| Picotements | Note moyenne sur 10 à J0 | 2,95 | 3,62 | 2,25 |
| | Note moyenne sur 10 à J30 | 0,59 | 0,68 | 0,50 |
| | *% évolution de J0 à J30* | *-80%* | *-81%* | *-78%* |
| Sensation de brûlure | Note moyenne sur 10 à J0 | 3,16 | 3,97 | 2,33 |
| | Note moyenne sur 10 à J30 | 0,49 | 0,57 | 0,42 |
| | *% évolution de J0 à J30* | -84% | *-86%* | -82% |
| Douleur | Note moyenne sur 10 à J0 | 0,81 | 0,97 | 0,64 |
| | Note moyenne sur 10 à J30 | 0,07 | 0,05 | 0,08 |
| | *% évolution de J0 à J30* | *-92%* | -94% | *-87%* |
| **Appréciation globale du dermatologue (*note moyenne sur 10*)** | | | | |
| Amélioration des signes objectifs | | *6,11* | *6,36* | 6,23 |
| Amélioration des symptômes subjectifs | | *6,3* | *6,75* | *6,52* |
| Produit adapté au soin de la peau sensible, irritée ou allergique | | *6,38* | *7,14* | *6,75* |

Ces différents résultats montrent que ladite crème de jour améliore sensiblement l'état des peaux hypersensibles, irritées ou à prédisposition allergique.

Ladite crème, formulée sans parfum ni colorant, assure ainsi une hydratation efficace des couches supérieures de l'épiderme et apporte une réponse adaptée aux peaux hyper sensibles, irritées ou à prédisposition allergique.

De plus, la crème testée a été évaluée par les consommatrices, très agréable d'utilisation, avec de bonnes qualités cosmétiques, telles qu'une texture agréable, un toucher non gras, une sensation de confort après application. Au point de vue de l'efficacité les volontaires percevaient une diminution de la réactivité de la peau aux environnement agressifs polluants ou desséchants, une baisse des irritations et des symptômes subjectifs de la peau sensible, une amélioration de la tolérance aux produits cosmétiques (produits lavants), un effet protecteur et apaisant immédiat et à long terme (75% de la population), une baisse des poussées d'inconfort (73%), une amélioration du seuil de tolérance (70%). Globalement, 89% d'entre elles sont satisfaites de la crème testée.

En conclusion, il a ainsi été montré de manière tout à fait surprenante, que l'OX100 en association avec LU105 inhibe presque totalement la migration des CD vers les GL. Par ailleurs, OX100 et LU105 agissent en synergie pour inhiber la migration des CD chez la souris sensibilisée.

Il a ainsi aussi été montré de manière tout à fait surprenante, que les mêmes proportions en OX100 et en LU105, incorporées dans une crème cosmétique, permettent d'assurer une hydratation efficace des couches supérieures de l'épiderme et d'apporter une réponse adaptée aux peaux hyper sensibles, irritées ou à prédisposition allergique

## Revendications

1. Utilisation d'un composé actif pour inhiber la migration des cellules de Langerhans qui est une oxazoline répondant aux formules générales suivantes: 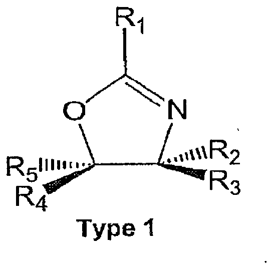 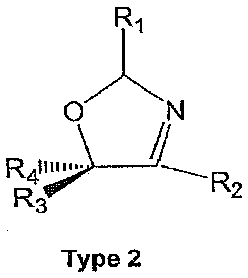 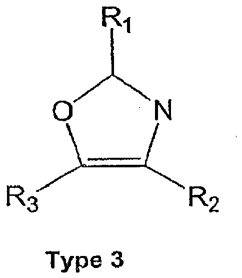 dans laquelle R₁ représente un groupe alkyle en C₁-C₄₀, linéaire ou ramifié, saturé ou insaturé, comprenant éventuellement une ou plusieurs insaturation(s) éthylénique(s) ainsi que un ou plusieurs substituant(s) choisi(s) dans le groupe formé par les radicaux hydroxy (OH) et alcoxy en C₁-C₆ (OC₁-C₆) ; R₂, R₃, R₄ et R₅ représentent, de manière indépendante, un atome d'hydrogène, un radical hydroxy, ou un groupe alkyle en C₁-C₃₀, linéaire ou ramifié, saturé ou insaturé, comprenant éventuellement une ou plusieurs insaturations éthyléniques ainsi que un ou plusieurs substituant(s) choisi(s) dans le groupe formé par les radicaux hydroxy (OH), alcoxy en C₁-C₆ (OC₁-C₆) et alcoxy en C₁-C₆ carbonyles (COOC₁-C₆) ;
pour l'obtention d'un médicament destiné au traitement ou la prévention des réactions allergiques, et/ou inflammatoires, et/ou irritatives de la peau et/ou des muqueuses.

2. Utilisation selon la revendication 1, **caractérisée en ce que** le médicament est destiné au traitement ou à la prévention des peaux et/ou des muqueuses sensibles et/ou réactives et/ou intolérantes et/ou inconfortables et/ou présentant un trouble de la barrière cutanée et/ou présentant un déséquilibre immunologique lié au vieillissement intrinsèque, extrinsèque ou hormonal.

3. Utilisation selon la revendication 1, **caractérisée en ce que** le médicament est destiné au traitement ou à la prévention de l'eczéma atopique et/ou de contact, des dermatoses inflammatoires telles que le psoriasis, des dermites irritatives, des maladies auto-immunes, de la photo-immuno-suppression ou du rejet de greffe.

4. Utilisation selon la revendication 1, **caractérisée en ce que** le médicament est destiné à diminuer le caractère allergisant et/ou irritant d'une composition ou d'un parfum.

5. Utilisation selon la revendication 1, **caractérisée en ce que** le médicament est destiné à prévenir et/ou traiter les peaux et/ou des muqueuses sensibles, irritées, intolérantes, à tendance allergique, âgées, soumises à un signal danger, présentant un trouble de la barrière cutanée, présentant des rougeurs cutanées ou présentant un déséquilibre immunologique non pathologique lié au vieillissement intrinsèque, extrinsèque ou hormonal.

6. Utilisation selon l'une quelconque des revendications précédentes, **caractérisée en ce que** le médicament comprend en outre un agent anti-inflammatoire non-stéroïdien.

7. Utilisation selon l'une quelconque des revendications précédentes, **caractérisée en ce que** la dite oxazoline est une oxazoline de type 1 sélectionnée dans le groupe composé de la 2-undécyl-4-hydroxyméthyl-4-méthyl-1,3-oxazoline, de la 2-undécyl-4,4-diméthyl-1,3-oxazoline, de la (E)-4,4-diméthyl-2-heptadéc-8-ényl-1,3-oxazoline, de la 4-hydroxyméthyl-4-méthyl-2-heptadécyl-1,3-oxazoline, la (E)-4-hydroxyméthyl-4-méthyl-2-heptadéc-8-ényl-1,3-oxazoline, la 2-undécyl-4-éthyl-4-hydroxyméthyl-1,3-oxazoline.

8. Utilisation selon l'une quelconque des revendications précédentes, **caractérisée en ce que** la dite oxazoline est la 2-undécyl-4,4-diméthyl-1,3-oxazoline appelée OX-100 de formule:

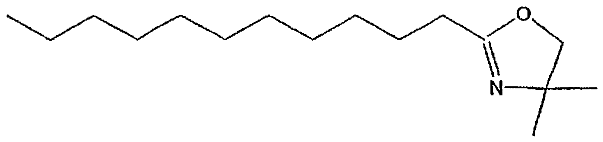

9. Utilisation selon l'une quelconque des revendications précédentes **caractérisée en ce que** la concentration en oxazoline est comprise entre environ 0,001 et environ 10 % en poids, par rapport au poids total du médicament.

10. Utilisation selon l'une quelconque des revendications précédentes, **caractérisée en ce que** le médicament comprend en outre
▪ un inhibiteur de métalloprotéases (MMPs) sélectionné dans le groupe constitué par
∘ les inhibiteurs tissulaires de métalloprotéinases choisis dans le groupe constitué de TIMP-1 et TIMP-2,
∘ l'alpha-2-macroglobuline,
∘ la bryostatine-1,
∘ les antibiotiques,
∘ les peptides synthétiques ou naturels ayant une structure similaire aux substrats des MMPs choisis dans le groupe constitué par le batimastat et le marimastat,
∘ les rétinoïdes,
∘ les antioxydants,
∘ les hydrolysats de malt,
∘ les extraits d'algues marines,
∘ les extraits de cartilage de requin et
∘ les extraits peptidiques de lupin
▪ les inhibiteurs de PKC choisis dans le groupe constitué par les composés phénoliques et polyphénoliques, les procyanidines, l'alpha-amyrine, le lupéol, le lupéol linoléate, les stérols, les stanols, les alcools triterpéniques et leurs homologues hydrogénés, les antibiotiques, Ro-318425 (ou 2-(8)-(aminométhyl)-6,7,8,9-tétrahydropyridol(1,2-a)indol-3-yl)3-(1-méthyl-indol-3-ylmaléimide, HCl), le diacylglycérol et le phorbol ester, les (lyso)sphingolipides, les lysophospholipides, les sphingosines et les phytosphingosines, les sphinganines,
▪ les agents apaisants choisis dans le groupe constitué par les dérivés de réglisse et les dérivés de l'alpha-bisabolol,
▪ les immunosuppresseurs,
▪ les chélateurs d'ions choisis dans le groupe constitué par l'acide éthylènediamine-tétracétique (EDTA) et ses sels de sodium, de potassium, de calcium disodium, de diammonium, de triéthanololamine (TEA-EDTA), l'acide hydroxyéthyl éthylène diamine tétracétique (HEDTA) et son sel trisodique, l'acide diéthylènetriamine pentacétique (DTPA), et leurs mélanges
▪ les chélateurs d'ions biologiques choisis dans le groupe constitué par la métallothionéine, la transférine, la lactoférine, la calmoduline, le chitosane méthylène phosphonate, et leurs mélanges
▪ les alcanolamides,
▪ les oxazolidinones et
▪ les dérivés d'acide carbamique répondant aux formules générales suivantes : 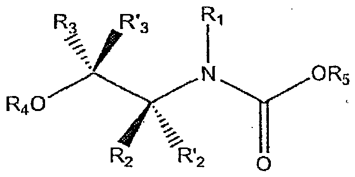
dans laquelle :
R₁ représente un atome d'hydrogène ou un groupe alkyle en C₁-C₃₀, linéaire ou ramifié, saturé ou insaturé, comprenant éventuellement une ou plusieurs insaturation(s) éthylénique(s) et/ou acétylénique(s), ainsi que un ou plusieurs substituant(s) hydroxy (OH);
R₂ et R'₂ représentent, de manière indépendante, un atome d'hydrogène ou un groupe alkyle en C₁-C₃₀, linéaire ou ramifié, saturé ou insaturé, comprenant éventuellement une ou plusieurs insaturation(s) éthylénique(s) et/ou acétylénique(s), ainsi que un ou plusieurs substituant(s) hydroxy (OH);
R₃ et R'₃ représentent, de manière indépendante, un atome d'hydrogène ou un groupe alkyle en C₁-C₃₀, linéaire ou ramifié, saturé ou insaturé, comprenant éventuellement une ou plusieurs insaturation(s) éthylénique(s) et/ou acétylénique(s), ainsi que un ou plusieurs substituant(s) hydroxy (OH);
R₄ et R₅ représentent, de manière indépendante, un atome d'hydrogène ou un groupe acyle de type RxCO, dans lequel Rx est un radical alkyle en C₁-C₃₀, linéaire ou ramifié, saturé ou insaturé, comprenant éventuellement une ou plusieurs insaturation(s) éthylénique(s) et/ou acétylénique(s), ainsi que un ou plusieurs substituant(s) hydroxy (OH).

11. Utilisation selon la revendication 10, **caractérisée en ce que** ledit inhibiteur des MMPs est choisi dans le groupe constitué par les extraits peptidiques de lupin, de préférence l'extrait B (LU105).

12. Utilisation selon la revendication 10, **caractérisée en ce que** ledit inhibiteur des MMPs est choisi dans le groupe constitué par les rétinoïdes.

## Patentansprüche

1. Verwendung einer aktiven Verbindung zur Hemmung der Migration der Langerhans-Zellen, die ein Oxazolin ist, das den folgenden allgemeinen Formeln entspricht: 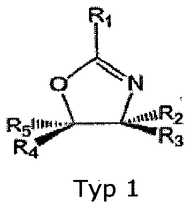 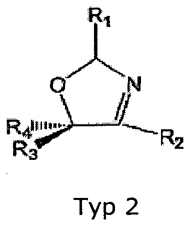 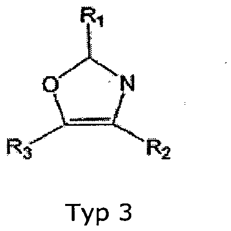 worin R₁ eine lineare oder verzweigte, gesättigte oder ungesättigte C₁-C₄₀-Alkylgruppe darstellt, gegebenenfalls enthaltend eine oder mehrere Ethylen-Unsättigungen sowie einen oder mehrere Substituenten, ausgewählt aus der Gruppe gebildet durch das Hydroxyl-Radikal (OH) und C₁-C₆-Alkoxylradikale (OC₁-C₆); worin R₂, R₃, R4 und R₅, voneinander unabhängig, ein Wasserstoffatom, ein Hydroxyl-Radikal oder eine lineare oder verzweigte, gesättigte oder ungesättigte C₁-C₃₀-Alkylgruppe darstellen, gegebenenfalls enthaltend eine oder mehrere Ethylen-Unsättigungen sowie einen oder mehrere Substituenten, ausgewählt aus der Gruppe gebildet durch das Hydroxyl-Radikal (OH), C₁-C₆-Alkoxylradikale (OC₁-C₆) und C₁-C₆-Alkoxycarbonylradikale (COOC₁-C₆);
zur Gewinnung eines Arzneimittels, das für die Behandlung oder die Vorbeugung allergischer und/oder entzündlicher und/oder irritativer Reaktionen der Haut und/oder der Schleimhäute bestimmt ist.

2. Verwendung nach Anspruch 1, **dadurch gekennzeichnet, dass** das Arzneimittel für die Behandlung oder die Vorbeugung empfindlicher und/oder reaktiver und/oder intoleranter und/oder unbequemer Haut und/oder Schleimhäute oder von Haut und/oder Schleimhäuten, und/oder die eine Störung der Hautbarriere und/oder ein immunologisches Ungleichgewicht aufweisen, das mit der altersbedingten, durch äußere Einwirkungen ausgelösten oder hormonellen Alterung verbunden ist, bestimmt ist.

3. Verwendung nach Anspruch 1, **dadurch gekennzeichnet, dass** das Arzneimittel für die Behandlung oder die Vorbeugung des atopischen Ekzems und/oder Kontaktekzems, entzündlicher Dermatosen wie Psoriasis, irritativer Dermatitis, von Autoimmunerkrankungen, der Photoimmunsuppression oder der Transplantatabstoßung bestimmt ist.

4. Verwendung nach Anspruch 1, **dadurch gekennzeichnet, dass** das Arzneimittel für die Verringerung des allergisierenden und/oder reizenden Charakters einer Zusammensetzung oder eines Parfums bestimmt ist.

5. Verwendung nach Anspruch 1, **dadurch gekennzeichnet, dass** das Arzneimittel für die Vorbeugung oder die Behandlung von Haut und/oder Schleimhäuten, die empfindlich, gereizt oder intolerant ist bzw. sind, zu Allergien neigt bzw. neigen, älter ist bzw. sind, einem Gefahrensignal ausgesetzt ist bzw. sind, eine Störung der Hautbarriere aufweist bzw. aufweisen, Hautrötungen aufweist bzw. aufweisen oder ein nicht pathologisches immunologisches Ungleichgewicht aufweist bzw. aufweisen, das mit der altersbedingten, durch äußere Einwirkungen ausgelösten oder hormonellen Alterung verbunden ist, bestimmt ist.

6. Verwendung nach einem der vorhergehenden Ansprüche, **dadurch gekennzeichnet, dass** das Arzneimittel ferner einen nichtsteroidalen entzündungshemmenden Wirkstoff enthält.

7. Verwendung nach einem der vorhergehenden Ansprüche, **dadurch gekennzeichnet, dass** das Oxazolin ein Oxazolin Typ 1 ist, ausgewählt aus der Gruppe bestehend aus 2-Undecyl-4-hydroxymethyl-4-methyl-1,3-oxazolin, 2-Undecyl-4,4-dimethyl-1,3-oxazolin, (E)-4,4-Dimethyl-2-heptadec-8-enyl-1,3-oxazolin, 4-Hydroxymethyl-4-methyl-2-heptadecyl-1,3-oxazolin, (E)-4-Hydroxymethyl-4-methyl-2-heptadec-8-enyl-1,3-oxazolin, 2-Undecyl-4-ethyl-4-hydroxymethyl-1,3-oxazolin.

8. Verwendung nach einem der vorhergehenden Ansprüche, **dadurch gekennzeichnet, dass** das Oxazolin 2-Undecyl-4,4-dimethyl-1,3-oxazolin, bezeichnet als OX-100, der folgenden Formel ist:

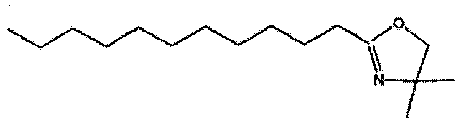

9. Verwendung nach einem der vorhergehenden Ansprüche, **dadurch gekennzeichnet, dass** die Konzentration an Oxazolin zwischen etwa 0,001 und etwa 10 Gew.-% bezogen auf das Gesamtgewicht des Arzneimittels beträgt.

10. Verwendung nach einem der vorhergehenden Ansprüche, **dadurch gekennzeichnet, dass** das Arzneimittel ferner Folgendes enthält:
• einen Metalloproteinase-Inhibitor (MMP), ausgewählt aus der Gruppe bestehend aus:
∘ den gewebespezifischen Metalloproteinase-Inhibitoren, ausgewählt aus der Gruppe bestehend aus TIMP-1 und TIMP-2,
∘ alpha-2-Makroglobulin,
∘ Bryostatin-1,
∘ den Antibiotika,
∘ den synthetischen oder natürlichen Peptiden mit einer ähnlichen Struktur wie die Substrate der MMPs, ausgewählt aus der Gruppe bestehend aus Batimastat und Marimastat,
∘ den Retinoiden,
∘ den Antioxidantien,
∘ den Malz-Hydrolysaten,
∘ den Meeresalgen-Extrakten,
∘ den Haifischknorpel-Extrakten und
∘ den Lupinenpeptid-Extrakten
• PKC-Inhibitoren, ausgewählt aus der Gruppe bestehend aus den Phenol- und Polyphenolverbindungen, den Procyanidinen, Alpha-Amyrin, Lupeol, Lupeollinoleat, den Sterolen, den Stanolen, den Triterpenalkoholen und ihren hydrogenierten Homologen, den Antibiotika, Ro318425 (oder 2-(8)-(Aminomethyl)-6,7,8,9-tetrahydropyridol(1,2-a)indol-3-yl)3-(1-methyl-indol-3-ylmaleimid, HCI), Diacylglycerol und Phorbolester, den (Lyso-) Sphingolipiden, den Lysophospholipiden, den Sphingosinen und den Phytosphingosinen, den Sphinganinen,
• beruhigende Wirkstoffe, ausgewählt aus der Gruppe bestehend aus den Süßholzderivaten und den alpha-Bisabolol-Derivaten,
• Immunosuppressoren,
• Ionen-Chelatbildner, ausgewählt aus der Gruppe bestehend aus Ethylendiamintetra-Essigsäure (EDTA) und ihren Natriumsalzen, Kaliumsalzen, Calciumdinatriumsalzen, Diammoniumsalzen, Triethanolaminsalzen (TEA-EDTA), Hydroxyetylethylendiamintetra-Essigsäure (1-HEDTA) und deren Trinatriumsalz, Diethylentriaminpentaessigsäure (DTPA) und deren Gemischen,
• biologische Ionen-Chelatbildner, ausgewählt aus der Gruppe bestehend aus Metallothionein, Transferrin, Lactoferrin, Calmodulin, Chitosanmethylenphosphonat und deren Gemischen
• Alkanolamid,
• Oxazolidinone und
• Carbamidsäure-Derivate, die den folgenden allgemeinen Formeln entsprechen: 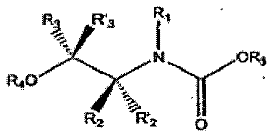 worin:
R₁ ein Wasserstoffatom oder eine lineare oder verzweigte, gesättigte oder ungesättigte C₁-C₃₀-Alkylgruppe darstellt, gegebenenfalls enthaltend eine oder mehrere Ethylen- und/oder Acetylen-Unsättigungen, sowie einen oder mehrere Hydroxyl-Substituenten (OH);
R₂ und R'₂, voneinander unabhängig, ein Wasserstoffatom oder eine lineare oder verzweigte, gesättigte oder ungesättigte C₁-C₃₀-Alkylgruppe darstellen, gegebenenfalls enthaltend eine oder mehrere Ethylen-und/oder Acetylen-Unsättigungen, sowie einen oder mehrere Hydroxyl-Substituenten (OH);
R₃ und R'₃, voneinander unabhängig, ein Wasserstoffatom oder eine lineare oder verzweigte, gesättigte oder ungesättigte C₁-C₃₀-Alkylgruppe darstellen, gegebenenfalls enthaltend eine oder mehrere Ethylen-und/oder Acetylen-Unsättigungen, sowie einen oder mehrere Hydroxyl-Substituenten (OH);
R₄ und R₅, voneinander unabhängig, ein Wasserstoffatom oder eine Acylgruppe des Typs RxCO darstellen, worin Rx ein lineares oder verzweigtes, gesättigtes oder ungesättigtes C₁-C₃₀-Alkylradikal darstellt, gegebenenfalls enthaltend eine oder mehrere Ethylen- und/oder Acetylen-Unsättigungen, sowie einen oder mehrere Hydroxyl-Substituenten (OH).

11. Verwendung nach Anspruch 10, **dadurch gekennzeichnet, dass** der MMP-Inhibitor ausgewählt ist aus der Gruppe bestehend aus den Lupinenpeptid-Extrakten, bevorzugt dem B-Extrakt (LU105).

12. Verwendung nach Anspruch 10, **dadurch gekennzeichnet, dass** der MMP-Inhibitor ausgewählt ist aus der Gruppe bestehend aus den Retinoiden.

## Claims

1. Use of an active compound for inhibiting the migration of Langerhans cells which is an oxazoline complying with the following general formulas: 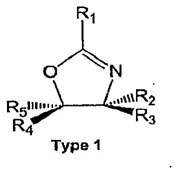 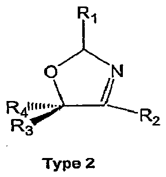 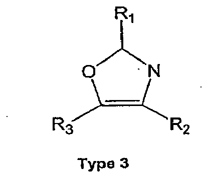 wherein R₁ represents a linear or branched, saturated or unsaturated C₁-C₄₀ alkyl group, optionally comprising one or a plurality of ethylenic unsaturation(s) along with one or a plurality of substituent(s) chosen from the group formed by the hydroxy (OH) and C₁-C₆ alkoxy (OC₁-C₆) radicals; R₂, R₃, R₄ and R₅ represent, independently, a hydrogen atom, a hydroxy radical, or a linear or branched, saturated or unsaturated, C₁-C₃₀ alkyl group, optionally comprising one or a plurality of ethylenic unsaturation(s) along with one or a plurality of substituent(s) chosen from the group formed by the hydroxy (OH) and C₁-C₆ alkoxy (OC₁-C₆) and C₁-C₆ alkoxy carbonyl (COOC₁-C₆) radicals;
to obtain a medicinal product intended for the treatment or prevention of allergic and/or inflammatory and/or irritant reactions of the skin and/or mucosa.

2. Use according to claim 1, **characterised in that** the medicinal product is intended for the treatment or prevention of skin and/or mucosa that are sensitive and/or reactive and/or intolerant and/or uncomfortable and/or having a skin barrier disorder and/or having an immunological imbalance linked with intrinsic, extrinsic or hormonal ageing.

3. Use according to claim 1, **characterised in that** the medicinal product is intended for the treatment or prevention of atopic and/or contact eczema, inflammatory dermatoses such as psoriasis, irritant dermatitis, autoimmune diseases, photo-immunosuppression or transplant rejection.

4. Use according to claim 1, **characterised in that** the medicinal product is intended to reduce the allergenic and/or irritant nature of a composition or a fragrance.

5. Use according to claim 1, **characterised in that** the medicinal product is intended to prevent and/or treat skin and/or mucosa that are sensitive, irritated, intolerant, prone to allergy, aged, subject to a danger signal, having a skin barrier disorder, having skin rashes or having non-pathological immunological imbalance linked with intrinsic, extrinsic or hormonal ageing.

6. Use according to any of the above claims, **characterised in that** the medicinal product further comprises a non-steroidal anti-inflammatory agent.

7. Use according to any of the above claims, **characterised in that** said oxazoline is a type 1 oxazoline selected from the group consisting of 2-undecyl-4-hydroxymethyl-4-methyl-1,3-oxazoline, 2-undecyl-4,4-dimethyl-1,3-oxazoline, (E)-4,4-dimethyl-2-heptadec-8-enyl-1,3-oxazoline, 4-hydroxymethyl-4-metyl-2-heptadecyl-1,3-oxazoline, (E)-4-hydroxymethyl-4-methyl-2-heptadec-8-enyl-1,3-oxazoline, 2-undecyl-4-ethyl-4-hydroxymethyl-1,3-oxazoline.

8. Use according to any of the above claims, **characterised in that** said oxazoline is 2-undecyl-4,4-dimethyl-1,3-oxazoline named OX-100 having the formula:

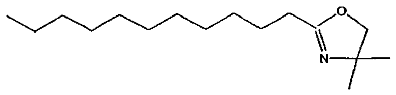

9. Use according to any of the above claims, **characterised in that** the oxazoline concentration is between approximately 0.001 and approximately 10% by weight, with respect to the total weight of the medicinal product.

10. Use according to any of the above claims, **characterised in that** the medicinal product further comprises
▪ an inhibitor of metalloproteinases (MMPs) selected from the group consisting of
o tissue inhibitors of metalloproteinases chosen from the group consisting of TIMP-1 and TIMP-2,
o alpha-2-macroglobulin,
o bryostatin-1,
o antibiotics,
o synthetic or natural peptides having a similar structure to the MMP substrates chosen from the group consisting of batimastat and marimastat,
o retinoids,
o antioxidants,
o malt hydrolysates,
o seaweed extracts,
o shark cartilage extracts and
o lupin peptide extracts
▪ PKC inhibitors chosen from the group consisting of phenol and polyphenol compounds, procyanidins, alpha-amyrin, lupeol, lupeol linoleate, sterols, stanols, triterpenic alcohols and the hydrogenated homologues thereof, antibiotics, Ro-318425 (or 2-(8)-(aminomethyl)-6,7,8,9-tetrahydropyridol(1,2-a)indol-3-yl)3-(1-methyl-indol-3-ylmaleimide, HCl), diacylglycerol and phorbol ester, (lyso)sphingolipids, lysophospholipids, sphingosines and phytosphingosines, sphinganines,
▪ soothing agents chosen from the group consisting of liquorice derivatives and alpha-bisabolol derivatives,
▪ immunosuppressants,
▪ ion chelators chosen from the group consisting of ethylenediamine-tetraacetic acid (EDTA) and the sodium, potassium, calcium disodium, diammonium, triethanolamine (TEA-EDTA) salts thereof, hydroxyethyl ethylene diamine tetraacetic acid (HEDTA) and the trisodium salt thereof, diethylenetriamine pentaacetic acid (DTPA), and mixtures thereof
▪ biological ion chelators chosen from the group consisting of metallothionein, transferrin, lactoferrin, calmodulin, chitosan methylene phosphonate, and mixtures thereof
▪ alkanolamides,
▪ oxazolidinones and
▪ carbamic acid derivatives complying with the following general formulas: 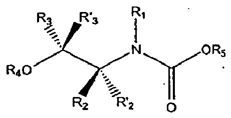
wherein:
R₁ represents a hydrogen atom or a linear or branched, saturated or unsaturated C₁-C₃₀ alkyl group, optionally comprising one or a plurality of ethylenic and/or acetylenic unsaturation(s), along with one or a plurality of hydroxy (OH) substituent(s);
R₂ and R'₂ represent, independently, a hydrogen atom or a linear or branched, saturated or unsaturated, C₁-C₃₀ alkyl group, optionally comprising one or a plurality of ethylenic and/or acetylenic unsaturation(s), along with one or a plurality of hydroxy (OH) substituent(s);
R₃ and R'₃ represent, independently, a hydrogen atom or a linear or branched, saturated or unsaturated, C₁-C₃₀ alkyl group, optionally comprising one or a plurality of ethylenic and/or acetylenic unsaturation(s), along with one or a plurality of hydroxy (OH) substituent(s);
R₄ and R₅ represent, independently, a hydrogen atom or an RxCO type group, wherein Rx is a linear or branched, saturated or unsaturated C₁-C₃₀ alkyl, optionally comprising one or a plurality of ethylenic and/or acetylenic unsaturation(s), along with one or a plurality of hydroxy (OH) substituent(s).

11. Use according to claim 10, **characterised in that** said inhibitor of MMPs is chosen from the group consisting of lupin peptide extracts, preferably extract B (LU105).

12. Use according to claim 10, **characterised in that** said inhibitor of MMPs is chosen from the group consisting of retinoids.
